# EUROPÄISCHE PATENTSCHRIFT

(11) **EP 0 937 094 B1**
(45) Veröffentlichungstag und Bekanntmachung des Hinweises auf die Patenterteilung: **03.04.2002**
(21) Anmeldenummer: 97913164.6
(22) Anmeldetag: 20.10.1997
(51) Int. Cl.: C07H 17/08

(54) **VERFAHREN ZUR HERSTELLUNG VON IVERMECTIN**
METHOD FOR THE PRODUCTION OF IVERMECTIN
PROCEDE DE FABRICATION D'IVERMECTINE

(30) Priorität: 31.10.1996 DE 19644050
(43) Veröffentlichungstag der Anmeldung: 25.08.1999
(73) Patentinhaber: BAYER AG, 51368 Leverkusen (DE)
(72) Erfinder: ARLT, Dieter, D-32657 Lemgo (DE); BONSE, Gerhard, D-51061 Köln (DE)
(86) Internationale Anmeldenummer: EP9705777
(87) Internationale Veröffentlichungsnummer: WO9818806

(56) Entgegenhaltungen:
- EP-A- 0 001 689
- EP-A- 0 059 616
- EP-A- 0 729 971

## Beschreibung

Die vorliegende Erfindung betrifft ein Verfahren zur Herstellung von Ivermectin durch selektive Hydrierung von Avermectin und anschließende Abtrennung des Katalysators.

Ivermectin ist eine bekannte Verbindung, die hervorragende biologische Wirkungen besitzt und als Anthelmintikum, Ektoparasitizid, Insektizid und Akarizid breite Verwendung findet.

Es ist bekannt (EP-A 0 001 689), Ivermectin durch selektive katalytische Hydrierung aus Avermectin B₁ₐ und B_{1b} herzustellen. Avermectin wird biotechnologisch mit Hilfe von Streptomyces avermitilis gewonnen. Es besitzt fünf Doppelbindungen. Um Ivermectin aus diesem Ausgangsmaterial herzustellen, benötigt man einen selektiv wirkenden Katalysator, der nur die 22,23-Doppelbindung hydriert. 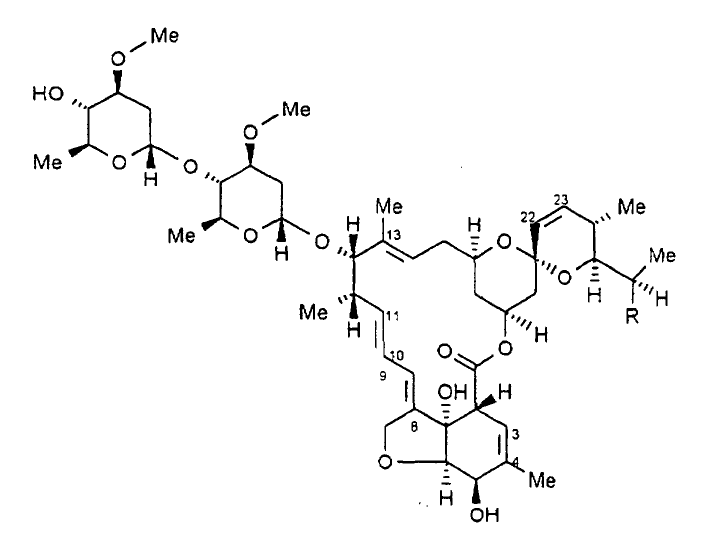 Avermectin B₁ₐ (R: -Ethyl)
Avermectin B_{1b} (R: -Methyl)

Es ist aus der EP-A 0 001 689 bekannt, Katalysatoren der allgemeinen formel [(R)₃P]₃RhX für diesen Zweck zu verwenden, bevorzugt wird der Wilkinson Katalysator [Ph₃P]₃RhCl eingesetzt. Dabei werden relativ große Mengen dieses Katalysators (0,05 bis 0,5 mol/mol Avermectin) verwendet, um die gewünschte Hydrierung zu erreichen.

Nach der Hydrierung ist es notwendig, das Edelmetall möglichst vollständig vom Produkt abzutrennen, um den Wirkstoff in einer spezifikationsgerechten Form (Schwermetallgehalt <10 ppm) zu erhalten.

Aus diesem Grund und wegen des hohen Rhodiumpreises wurde vorgeschlagen (EP-A 0 059 616), zur Abtrennung und Rückgewinnung der erheblichen Mengen dieses Edelmetalls, die zur Herstellung von Ivermectin verwendet werden müssen, einen besonderen Wiedergewinnungsprozeß einzusetzen.

Dieser in EP-A 59 616 beschriebene Prozess umfaßt die Behandlung der nach der Hydrierung erhaltenen Produktlösungen mit bestimmten organischen Schwefelverbindungen bei erhöhten Temperaturen, beispielsweise 95°C, über mehrere Stunden, gefolgt von einer Kühlung der erhaltenen Mischung auf 0 bis 5°C, nachfolgend eine Filtration der abgeschiedenen Rhodiumverbindungen und gegebenenfalls eine Extraktion der filtrierten organischen Rohlösung mit wäßriger Sodalösung zur weiteren Reinigung.

Diese Ausführung der Abtrennung des Katalysatormetalls ist zeit- und energieaufwendig, belastet zudem das empfindliche Produkt und beläßt die im Katalysator enthaltenen Liganden (Phosphine) im Produkt. Das Produkt wird bei dieser Vorgehensweise außerdem durch die Zugabe von Überschußmengen an organischen Schwefelverbindungen (5 mol/mol Rhodium) zusätzlich verunreinigt. Diese Komponenten müssen zur Herstellung des reinen Wirkstoffes durch verlustreiche Umkristallisation entfernt werden.

Gegenstand der vorliegenden Erfindung ist ein Verfahren zur Herstellung von Ivermectin, das es erlaubt, sowohl das eingesetzte Rhodium als auch die organischen Komponenten des Katalysatorsystems in einfacher Weise aus der nach der Hydrierung erhaltenen Produktlösung abzutrennen, um einen Wirkstoff zu erhalten, der unter geringen Materialverlusten in eine anwendungsgerechte Form verarbeitet werden kann.

Bei diesem Verfahren wird Avermectin B₁ₐ und B_{1b} hydriert und aus den erhaltenen Reaktionslösungen sowohl das Katalysatormetall als auch die organischen Komponenten des Katalysatorsystems in einfacher Weise abgetrennt.

Bei dem erfindungsgemäßen Verfahren werden Gemische von Avermectin B₁ₐ und B_{1b} durch selektive Hydrierung unter Verwendung von Katalysatoren, die in an sich bekannter Weise aus Rhodiumsalzen oder Rhodiumkomplexverbindungen und Phosphinen, gegebenenfalls unter Zusatz von Hydrazin oder Hydrazinsalzen erhalten werden,
wobei als Phosphine komplex-bildende Phosphine der Formel (I) 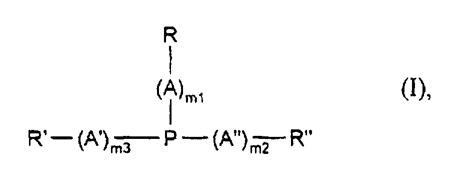 in welcher
- R, R' und R": unabhängig voneinander für Wasserstoff, Alkyl, oder gegebenenfalls durch Alkyl, Alkoxy, Halogen oder Halogenalkyl substituiertes Arylalkyl stehen,
- A, A' und A": unabhängig voneinander für gegebenenfalls durch Alkyl oder Alkoxy und/oder gegebenenfalls durch Halogen oder Halogenalkyl substituierte zweiwertige aromatische Reste stehen,
- m₁, m₂ und m₃: gleich oder verschieden sind und 0 oder 1 bedeuten,
wobei die Summe der in den Alkyl- und Alkoxygruppen enthaltenen Kohlenstoffatome mindestens 12 beträgt,
verwendet werden,
zu Ivermectin umgesetzt und nachfolgend das Katalysatorsystem aus dem erhaltenen Reaktionsgemisch, gegebenenfalls nach Entfernen des Lösungsmittels,
mit lipophilen Lösungsmitteln entfernt.

Die Herstellungsverfahren für die Katalysatoren sind bekannt (s. z.B. Inorg. Synth. 10, 67 (1967) und EP-A 0 086 040, EP-A 0 283 615 und Tetrahedron Vol. 7, No. 19/20, S. 2087-2089 (1988)). Die als Ausgangsverbindungen für die Herstellung der Katalysatoren infrage kommenden Rhodiumverbindungen sind bekannt; beispielsweise seien genannt als Rhodiumsalze Rhodium(III)-chlorid-Hydrat und Rhodium(III)-bromid-Hydrat, als geeignete Precursor aus der Reihe der Rhodiumkomplexverbindungen Rhodium(I)-(1c, 5c-cyclooctadien)-chlorid dimer, Rhodium(I)-(1,5-hexadien)-chlorid dimer und Rhodium(I)-(2,5-norbornadien)-chlorid dimer sowie Rhodium(I)-(1,5-cyclooctadien)acetylacetonat.

Die erfindungsgemäß zum Einsatz kommenden Phosphine der Formel (I) sind bekannt oder können nach bekannten Verfahren hergestellt werden (s. Houben-Weyl, Methoden der Organischen Chemie, 4. Aufl., Bd. XII/1, Georg Thieme Verlag Stuttgart, 1963).

Vorzugsweise werden in das erfindungsgemäße Verfahren Phosphine der Formel (I) eingesetzt, in welcher unabhängig voneinander
- R, R', R": für Wasserstoff oder C₁-C₂₀-Alkyl oder für gegebenenfalls durch C₁-C₂₀-Alkyl, C₁-C₂₀-Alkoxy, Halogen, insbesondere Chlor, Fluor, Brom, 1 - 5 Halogen-C₁-C₄-alkyl, insbesondere Trifluormethyl substituiertes Aryl-C₁-C₄-alkyl, insbesondere Benzyl oder Phenylethyl steht und in welcher unabhängig voneinander
- A, A', A": für einen gegebenenfalls durch C₁-C₂₀-Alkyl, C₁-C₂₀-Alkoxy, Halogen, insbesondere Fluor oder Chlor, 1 - 5 Halogen-C₁-C₄-alkyl, insbesondere Trifluormethyl substituierten zweiwertigen aromatischen Rest, insbesondere Phenyl stehen und in der
- m₁ und m₂ 1 und m₃ 0: bedeutet, wobei die Summe der in den Alkyl- und Alkoxygruppen enthaltenen Kohlenstoffatome mindestens 12, bevorzugt mindestens 15 und besonders bevorzugt mindestens 18 beträgt.

Besonders bevorzugt sind Phosphine der Formel (I), in welcher unabhängig voneinander
- R, R', R": für Wasserstoff oder C₁-C₂₀-Alkyl oder für gegebenenfalls durch C₁-C₂₀-Alkyl, C₁-C₂₀-Alkoxy, Halogen, insbesondere Chlor, Fluor, Brom, 1 - 5 Halogen-C₁-C₄-alkyl, insbesondere Trifluormethyl substituiertes Aryl-C₁-C₄-alkyl, insbesondere Benzyl oder Phenylethyl steht und in welcher unabhängig voneinander
- A, A', A": für einen gegebenenfalls durch C₁-C₂₀-Alkyl, C₁-C₂₀-Alkoxy, Halogen, insbesondere Fluor oder Chlor, 1 - 5 Halogen-C₁-C₄-alkyl, insbesondere Trifluormethyl substituierten zweiwertigen aromatischen Rest, insbesondere Phenyl stehen und in der
- m₁, m₂, m₃: für 1 stehen,
wobei die Summe der Kohlenstoffatome in den Alkyl- oder Alkoxy-Gruppen mindestens 12, bevorzugt mindestens 15 und besonders bevorzugt mindestens 18 beträgt.

Beispielsweise seien genannt:
(2-Dodecyl-phenyl)-diphenyl-phosphin, (3-Dodecylphenyl)-diphenyl-phosphin, (4-Dodecylphenyl)-diphenyl-phosphin, Bis-(4-tert.-butylphenyl)-(4-dodecyl)-phosphin, Tris-(4-tert.-butylphenyl)-phosphin, Bis-o-tolyl-(4-dodecylphenyl)-phosphin, (4-Octadecylphenyl)-diphenyl-phosphin, Dodecyl-diphenyl-phosphin, Bis-(dodecyl)-phenyl-phosphin, Methyl-bis-(dodecylphenyl)-phosphin, (4-Trifluormethylphenyl)-bis-(dodecyl)-phenyl-phosphin, (4-Octadecylphenyl)-bis-(4-chlorphenyl)-phosphin, Bis-(2-methoxyphenyl)-(4-dodecylphenyl)-phosphin, (4-Dodecyloxyphenyl)-diphenyl-phosphin, Dodecylbenzyl-diphenyl-phosphin, 4-Biphenyl-bis-(dodecylphenyl)-phosphin, Tris-(octylphenyl)-phosphin, Tris-(hexylphenyl)-phosphin, Tris-(nonylphenyl)-phosphin, Tris-(decylphenyl)-phosphin, Bis-(hexadecylphenyl)-phenyl-phosphin, Bis-(octadecylphenyl)-phenyl-phosphin.

Die Katalysatoren können nach der Herstellung isoliert und in reiner Form für die Hydrierung eingesetzt werden. Es ist aber auch möglich und besonders zweckmäßig, die Katalysatoren in situ zu synthetisieren und entsprechend erhaltene Lösungen für die selektive Hydrierung einzusetzen. Es kann von Vorteil sein, dem Hydrieransatz das als Ligand verwendete Phosphin in einem Überschuß zuzusetzen.

Zur Herstellung des Katalysatorsystems (vgl. insbesondere EP-A 0 086 046) werden Rhodiumsalze und Phosphine der Formel (I) im Molverhältnis 1:1 bis 1:20, bevorzugt 1:1 bis 1:15, besonders bevorzugt 1:3 bis 1:15 eingesetzt. Gegebenenfalls wird Hydrazin oder seine Derivate im Molverhältnis zu Rhodiumsalz 1:1 bis 1:10 zugesetzt.

Die beim erfindungsgemäßen Verfahren zugesetzte Menge an zusätzlichem Phosphin der Formel (I) liegt bezogen auf 1 mol Substrat in der Größenordnung von 0,01 bis 0,06 mol (vgl. EP-A 0 086 046). Die günstigste Menge kann jedoch leicht in einer Versuchsreihe ermittelt weren.

Die katalytische Hydrierung wird in üblichen Lösungsmitteln, wie z.B. Alkoholen, aromatischen Kohlenwasserstoffen, in Ethern, Ketonen, Estern oder in Mischungen von Lösungsmitteln, beispielsweise in Methanol- oder Aceton-Kohlenwasserstoff-Gemischen, durchgeführt.

Die Temperatur während der Hydrierung liegt im Bereich von ca. 40 bis 100°C, der Wasserstoffdruck im Bereich von etwa 1 bis 50 bar. Um die Reaktionszeit zu verkürzen, ist es zweckmäßig, bei Überdruck zu arbeiten, bevorzugt wird ein Bereich von 3 bis 20 bar.

Die lipophilen Eigenschaften der erfindungsgemäß als Katalysatorligand eingesetzten Phosphine ermöglichen eine einfache extraktive Abtrennung des Katalysatorsystems vom Produkt mit Hilfe geeigneter lipophiler Lösungsmittel, in denen das Verfahrensprodukt (Ivermectin) nicht oder nur geringfügig löslich ist.

So kann beispielsweise nach der Hydrierung das Lösungsmittel durch Vakuumdestillation und anschließend aus dem zurückbleibenden Produkt-Katalysator-Gemisch das Katalysatorsystem (Metallkomplex und Phosphin) durch Ausrühren mit lipophilen Lösungsmitteln entfernt werden. Das so erhaltene Ivermectin ist weitgehend frei von Katalysatormetall und Ligand-Phosphin und kann z.B. durch eine nachfolgende, an sich bekannte Umkristallisation, die der Abtrennung von geringen Mengen an Nebenprodukten dient, in hochreiner Form erhalten werden. Es ist auch möglich, nach der Entfernung des Katalysatorsystems das Produkt chromatografisch nahezu verlustfrei weiter aufzureinigen.

Zur selektiven Abtrennung des Katalysatorsystems eignen sich lipophile Lösungsmittel, wie z.B. aliphatische Kohlenwasserstoffe - beispielhaft genannt seien Cyclohexan, Methylcyclohexan, Isooctan, Petrolether, Waschbenzin oder Ether mit größeren Kohlenwasserstoffresten, wie z.B. tert.-Octylmethylether.

Eine Variante zur Abtrennung des Katalysatorsystems nach der Hydrierung besteht darin, daß man der Produkt-Katalysatorlösung, die nach der selektiven Hydrierung erhalten wird, das selektive, lipophile Lösungsmittel zusetzt und die polare, im Hydrieransatz erhaltene Lösungsmittelkomponente destillativ entfernt. Dabei tritt eine Entmischung ein, das Ivermectin fällt aus und kann von der Lösung, die das Katalysatorsystem enthält, durch Dekantieren oder Filtration getrennt werden.

Eine weitere Variante zur Abtrennung des Katalysatorsystems nach der Hydrierstufe besteht darin, daß man anschließend ein Zweiphasengemisch herstellt, das eine Separierung des Ivermectins vom Katalysatorsystem (Katalysator-Komplex und überschüssiges Phosphin) erlaubt. Dazu wird - falls notwendig - das für die Hydrierstufe eingesetzte Lösungsmittel destillativ, zweckmäßigerweise zur Schonung des Produktgemisches unter vermindertem Druck, entfernt und durch ein für die Separierung geeignetes Lösungsmittelgemisch ersetzt.

Für diese Variante der Separierung geeignete Lösungsmittelgemische bestehen aus einer lipophilen Komponente (s.o.) und aus polaren Lösungsmitteln, die mit Wasser mischbar sind, und Wasser. Als polare Komponenten für solche Lösungsmittelgemische seien beispielsweise genannt Methanol, Ethanol, Aceton, Butanon, Acetonitril, Tetrahydrofuran, Formamid, Dimethylformamid und N-Methylpyrrolidon. Der Wassergehalt solcher Lösungsmittelgemische kann je nach Auswahl der Komponenten unterschiedlich sein, er beträgt i.a. 5 bis 60 %, vorzugsweise 10 bis 40 %.

Es wurden gefunden, daß bei Vermischung eines solchen zweiphasigen Systems mit dem Rohprodukt der Hydrierstufe das Ivermectin ganz überwiegend in der polaren Komponente und das Katalysatorsystem ganz überwiegend in der lipophilen Komponente angereichert wird. Zweckmäßig ist für diese Ausführungsform die Abtrennung des Katalysatorsystems vom Produkt in einem kontinuierlichen Gegenstromverfahren unter Verwendung einer Extraktionskolonne.

Eine weitere Ausführung des erfindungsgemäßen Verfahrens besteht darin, Lösungsmittel bzw. Lösungsmittelgemische zu verwenden, die bei erhöhter Temperatur, die den Hydrierbedingungen entspricht, Edukt und Produkt sowie das Katalysatorsystem lösen und aus denen anschließend nach ausreichender Abkühlung das erwünschte Produkt (Ivermectin) ausfällt, in denen jedoch der Metallkomplex-Katalysator und das Ligandphosphin auch bei erniedrigter Temperatur gelöst bleiben. In diesem Falle kann das Verfahrensprodukt, weitgehend vom Katalysators befreit, durch Filtration von der Restlösung getrennt werden, während das Katalysatormetall in einfacher Weise durch deren destillative Aufarbeitung im Destillationsrückstand erhalten und der Wiederaufbereitung zugeführt wird.

Solche Lösungsmittel zeichnen sich durch amphiphile Eigenschaften aus und besitzen einen lipophilen Anteil sowie polare Gruppierungen. Genannt seien beispielhaft Isooctanol, Dodecanol, Methyl-tert.-octyl-ether, Gemische aus tert.-Butanol und Isooctan sowie tert.-Butylmethylether und Isooctan.

Es ist sehr überraschend, daß die erfindungsgemäß eingesetzten Katalysatoren bzw. Katalysatorsysteme sowohl eine hervorragend selektive Hydrierung von Avermectinen zu Ivermectinen als auch eine einfache Separierung von Produkt und Katalysatorsystem ermöglichen.

### Beispiele

### Beispiel 1

A) Katalysatorherstellung:
Eine Mischung von 7,5 mg Rhodiumtrichlorid, 30,9 mg Tris-(hexylphenyl)-phosphin, 3 ml Aceton und 15 µl Hydrazinhydrat wird unter Rühren und Rückflußkühlung unter Argonatmosphäre 4 Stunden erhitzt.

B) Hydrierung:
Die nach (A) erhaltene Katalysatorlösung wird zu einer Lösung von 4,3 g Avermectin (B₁ₐ- und B_{1b}-Gemisch) in 25 ml eines Gemisches von Aceton und Cyclohexan im Verhältnis 2:1 zugegeben. Nach Zusatz von 51,4 mg Tris-(hexylphenyl)-phosphin wird die Hydrierung in einem Stahlautoklaven bei 5 bar Wasserstoffdruck und 88°C ausgeführt. Nach 4 Stunden Hydrierzeit ergibt eine HPLC-Analyse einen Gehalt von 8,9 % Ausgangsmaterial, 89,9 % Ivermectin (B₁ₐ- und B_{1b}-Gemisch), Gehalt an Tetrahydroavermectin <0,1 %.

D) Abtrennung des Katalysatorsystems
Das nach (B) erhaltene Rohprodukt wird nach destillativem Entfernen des Lösungsmittelgemisches in einem Gemisch von 35 ml Methanol und 20 ml Wasser gelöst, diese Lösung wird mit 25 ml Cyclohexan in einem Schütteltrichter extrahiert. Die Phasen werden getrennt und im Vakuum eingeengt. Die Extraktion wird in der gleichen Weise zweimal wiederholt.

| | | |
|---|---|---|
| Ergebnis: | Das Rohprodukt der Hydrierstufe enthält | 690 ppm Rh |
| | | |

| | Das erhaltene Produkt | |
|---|---|---|
| | nach der 1. Extraktion | 39 ppm Rh |
| | nach der 2. Extraktion | 29 ppm Rh |
| | nach der 3. Extraktion | 22 ppm Rh |

Das aus dem Produkt extrahierte Katalysatorsystem (Katalysator und Phosphin) enthält 6.332 ppm Rh.

### Beispiel 2

A) Katalysatorherstellung:
   Eine Mischung von 7,5 mg Rhodiumtrichloridhydrat, 45,6 mg Tris-(octylphenyl)-phosphin (94%ig), 3 ml Aceton und 15 µl Hydrazinhydrat wird unter Rühren und Rückflußkühlung unter Argonatmosphäre 4 Stunden erhitzt.
B) Hydrierung:
   4,3 g Avermectin (B₁ₐ- und B_{1b}-Gemisch) werden nach Zusatz von 53,2 mg Tris-(octylphenyl)-phosphin unter den in Beispiel 1 (B) angegebenen Bedingungen hydriert. Nach 7 Stunden Hydrierzeit wird ein Ivermectinrohprodukt erhalten, das nach HPLC-Analyse 1,3 % Avermectin, 94,8 % Ivermectin und 2 % Tetrahydroavermectin enthält.
C) Abtrennung des Katalysatorsystems:
   Das erhaltene Produkt wird analog Beispiel 1 (C) behandelt. Nach der dritten Extraktion wird ein Produkt erhalten, das einen Gehalt von 9 ppm Rhodium aufweist.

## Patentansprüche

1. Verfahren zur Herstellung von Ivermectin, wobei Gemische von Avermectin B₁ₐ und B_{1b} durch selektive Hydrierung unter Verwendung von Katalysatoren, die in an sich bekannter Weise aus Rhodiumsalzen oder Rhodiumkomplexverbindungen und Phosphinen, gegebenenfalls unter Zusatz von Hydrazin oder Hydrazinsalzen erhalten werden,
wobei als Phosphine komplex-bildende Phosphine der Formel (I) 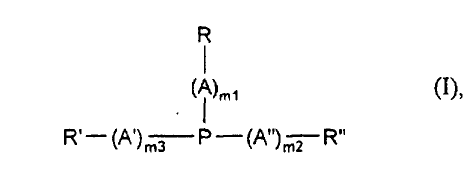 in welcher
R, R' und R" unabhängig voneinander für Wasserstoff, Alkyl, oder gegebenenfalls durch Alkyl, Alkoxy, Halogen oder Halogenalkyl substituiertes Arylalkyl stehen,
A, A' und A" unabhängig voneinander für gegebenenfalls durch Alkyl oder Alkoxy und/oder gegebenenfalls durch Halogen oder Halogenalkyl substituierte zweiwertige aromatische Reste stehen,
m₁, m₂ und m₃ gleich oder verschieden sind und 0 oder 1 bedeuten,
wobei die Summe der in den Alkyl- und Alkoxygruppen enthaltenen Kohlenstoffatome mindestens 12 beträgt,
verwendet werden,
zu Ivermectin umgesetzt werden und nachfolgend das Katalysatorsystem aus dem erhaltenen Reaktionsgemisch, gegebenenfalls nach Entfernen des Lösungsmittels,
mit lipophilen Lösungsmitteln entfernt wird.

## Claims

1. Process for preparing ivermectin, **characterized in that** mixtures of avermectin B₁ₐ and B_{1b} are reacted by selective hydrogenation using catalysts which are obtained in a manner known per se from rhodium salts or from complex rhodium compounds and phosphines, if appropriate by adding hydrazine or hydrazine salts,
using complex-forming phosphines of the formula (I) 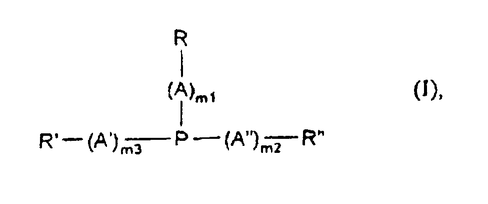 in which
R, R' and R" independently of one another each represent hydrogen, alkyl or optionally alkyl-, alkoxy-, halogen- or halogenoalkyl-substituted arylalkyl,
A, A' and A" independently of one another each represent optionally alky)- or alkoxy- and/or optionally halogen- or halogenoalkyl-substituted divalent aromatic radicals,
m₁, m₂ and m₃ are identical or different and are each 0 or 1,
as phosphines,
the sum of the carbon atoms present in the alkyl and alkoxy groups being at least 12,
to give ivermectin, and the catalyst system is subsequently removed from the resulting reaction mixture, if appropriate after removal of the solvent,
using lipophilic solvents.

## Revendications

1. Procédé d'hydrogénation d'ivermectine, dans lequel des mélanges d'avermectine B₁ₐ et B_{1b} sont transformés en ivermectine par hydrogénation sélective en recourant à des catalyseurs qui sont obtenus de manière connue en soi à partir de sels de rhodium ou de composés de complexe de rhodium et de phosphines, éventuellement avec utilisation d'hydrazine ou de sels d'hydrazine,
en utilisant, comme phosphines, des phosphines formant des complexes de formule (I) 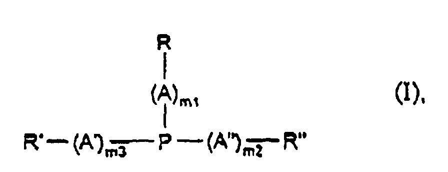 dans laquelle R, R' et R'' représentent indépendamment l'un de l'autre l'hydrogène, un alkyle ou éventuellement un arylalkyle substitué par un alkyle, un alkoxy, un halogène ou un halogénoalkyle,
A, A' et A'' représentent, indépendamment l'un de l'autre, des groupements aromatiques bivalents éventuellement substitués par un alkyle ou un alkoxy et/ou éventuellement par un halogène ou un halogénoalkyle,
m₁, m₂ et m₃, identiques ou différents, représentant 0 ou 1,
la somme des atomes de carbone contenus dans les groupes alkyle et alkoxy étant d'au moins 12,
et ensuite, le système de catalyseur est séparé du mélange de réaction obtenu, éventuellement après séparation du solvant, à l'aide de solvants lipophiles.
